# FASCICULE DE BREVET EUROPEEN

(11) **EP 2 328 624 B1**
(45) Date de publication et mention de la délivrance du brevet: **22.04.2020**
(21) Numéro de dépôt: 09802563.8
(22) Date de dépôt: 03.08.2009
(51) Int. Cl.: A61L 2/10

(54) **DISPOSITIF DE DÉCONTAMINATION À SOURCE LUMINEUSE ADAPTÉ AUX OBJETS PRÉSENTANT UNE CAVITÉ**
DEKONTAMINATIONSVORRICHTUNG MIT EINER LICHTQUELLE FÜR OBJEKTE MIT VERTIEFUNGEN
DECONTAMINATION DEVICE WITH A LIGHT SOURCE ADAPTED FOR OBJECTS HAVING A CAVITY

(30) Priorité: 01.08.2008 FR 0804380
(43) Date de publication de la demande: 08.06.2011
(73) Titulaire: Claranor, 84140 Montfavet (FR)
(72) Inventeur: LACOUR, Bernard, F-91240 Saint Michel sur Orge (FR)
(74) Mandataire: IPAZ
(86) Numéro de dépôt international: PCT/FR2009/000970
(87) Numéro de publication internationale: WO 2010/012915

(56) Documents cités:
- EP-A- 1 033 138
- WO-A-00/38740
- DE-A1- 3 108 854
- GB-A- 495 499
- US-A- 1 813 021
- US-A- 4 910 942
- US-A1- 2006 228 251

## Description

La présente invention concerne un dispositif de décontamination à source lumineuse adapté aux objets présentant une cavité.

Il est naturellement impératif de décontaminer les emballages dans lesquels sont stockés les produits alimentaires afin de garantir la qualité et la longévité de ces produits.

Ainsi une première technique connue consiste à pratiquer un traitement thermique du matériau destiné à être en contact avec les produits à conserver.

Un tel traitement est mal adapté aux matériaux de synthèse couramment utilisés aujourd'hui car ceux-ci présentent une résistance thermique très limitée, si bien qu'un traitement efficace au plan biologique conduit généralement à leur dégradation.

Il s'ensuit que les différentes industries traitant de problèmes de contamination emploient majoritairement une deuxième solution dont le principe est la désinfection chimique. Une première limitation de cette solution réside dans le fait que le traitement laisse des résidus sur le matériau d'emballage désinfecté et, par voie de conséquence, ces résidus sont transférés sur le produit alimentaire conservé dans l'emballage. La quantité de résidus produits est directement liée à la complexité géométrique de l'emballage. Une seconde limitation est due à la résistance de plus en plus importante des micro-organismes aux agents de désinfection. Une troisième limitation tient aux risques organoleptiques liés à la persistance d'une partie des agents chimiques employés à l'issue de la désinfection. Une quatrième limitation tient au caractère polluant de tout traitement chimique ; il convient en effet de traiter les effluents liquides au cours de la désinfection.

Par ailleurs, le procédé thermique et le procédé chimique sont pénalisants au niveau économique. Ils sont friands d'énergie, ils consomment une quantité d'eau non négligeable et ils requièrent des opérations de maintenance relativement lourdes.

Une troisième solution émerge qui tente de pallier les limitations des deux solutions précédentes.

Il s'agit d'une technique photonique qui assure la décontamination au moyen d'un flux lumineux. Cette technique de décontamination est décrite, par exemple, dans les documents GB495499, WO 00/38740, US 2006/0228251 A1, US1813021. Un premier type de source lumineuse est la lampe à vapeur de mercure. Celle-ci présente un rendement optique assez faible et elle délivre peu d'énergie dans la bande spectrale qui présente le maximum d'efficacité quant à la décontamination microbiologique, à savoir la bande 180 - 400 nanomètres. De plus, la lampe à mercure demande plusieurs minutes après son allumage pour fournir un flux lumineux stabilisé. Ce temps de latence est difficilement compatible avec certains procédés industriels et un fonctionnement en mode continu s'impose alors que ce n'est pas toujours la solution la plus appropriée. En outre, le fonctionnement continuel de la lampe pose des problèmes d'évacuation de chaleur lors des arrêts non programmés de l'installation de décontamination. On signalera encore le danger potentiel que présente le mercure tant pour l'environnement que pour le personnel de l'installation.

La lampe à mercure n'étant pas réellement satisfaisante pour les raisons mentionnées ci-dessus, la lampe à xénon moyenne pression fonctionnant en mode pulsé est apparue dans le domaine de la décontamination photonique. Ainsi le document EP 0 290 443 B1 propose des appareils fonctionnant avec une ou plusieurs lampes de ce deuxième type.

Un premier appareil comporte une lampe en forme d'hélice prévue pour illuminer un film souple conformé en cylindre de révolution.

Un deuxième appareil est destiné à la décontamination de corps creux, des conteneurs cylindriques en l'occurrence. Selon une première option, des lampes sont disposées au-dessus des conteneurs ; dans ce cas l'efficacité de l'illumination est très limitée car la plupart du flux lumineux n'atteint pas la cible, à savoir l'intérieur des conteneurs. Selon une seconde option, les lampes sont introduites dans les conteneurs ; dans ce cas l'efficacité de l'illumination est sensiblement améliorée.

Ce deuxième appareil n'est pas adapté à la décontamination externe des conteneurs. Pour réaliser cette décontamination externe, il conviendrait de prévoir une batterie de lampes additionnelles en charge de cette opération, l'efficacité et la fiabilité de l'illumination étant ici encore des plus réduite.

La présente invention a ainsi pour objet d'améliorer sensiblement l'efficacité et la fiabilité de l'illumination pour la décontamination d'un objet creux présentant une cavité tant dans cette cavité que sur une partie au moins de sa surface externe. La présente invention concerne un dispositif selon la revendication 1.

Ainsi, le réflecteur permet d'illuminer au moins en partie l'extérieur de l'objet.

De préférence, l'organe de positionnement comporte également des moyens pour produire un déplacement transversal relatif du support d'insolation par rapport à la source lumineuse, ce déplacement s'effectuant selon un axe distinct de leur axe commun.

Ceci afin de déplacer les objets à décontaminer.

Avantageusement, la plus grande dimension de la lampe coïncide avec l'axe commun.

Par souci d'efficacité, la source lumineuse est en service en position de travail et hors service en position de repos.

De plus, un moyen peut être installé pour refroidir cette source lumineuse.

Généralement, les surfaces extérieures des pièces à décontaminer présentent une symétrie de révolution.

De ce fait, la forme privilégiée du réflecteur est une calotte sphéroïdale.

Avantageusement, la lampe est une lampe à xénon moyenne pression fonctionnant en mode pulsé.

La lampe présente un corps rayonnant saillant.

La présente invention apparaîtra maintenant avec plus de détails dans le cadre de la description qui suit d'un exemple de réalisation donné à titre illustratif en se référant :
- la figure 1, un schéma du dispositif de décontamination en position de travail, et
- la figure 2, un schéma du dispositif de décontamination en position de repos.

Les éléments présents dans les deux figures sont affectés d'une seule et même référence.

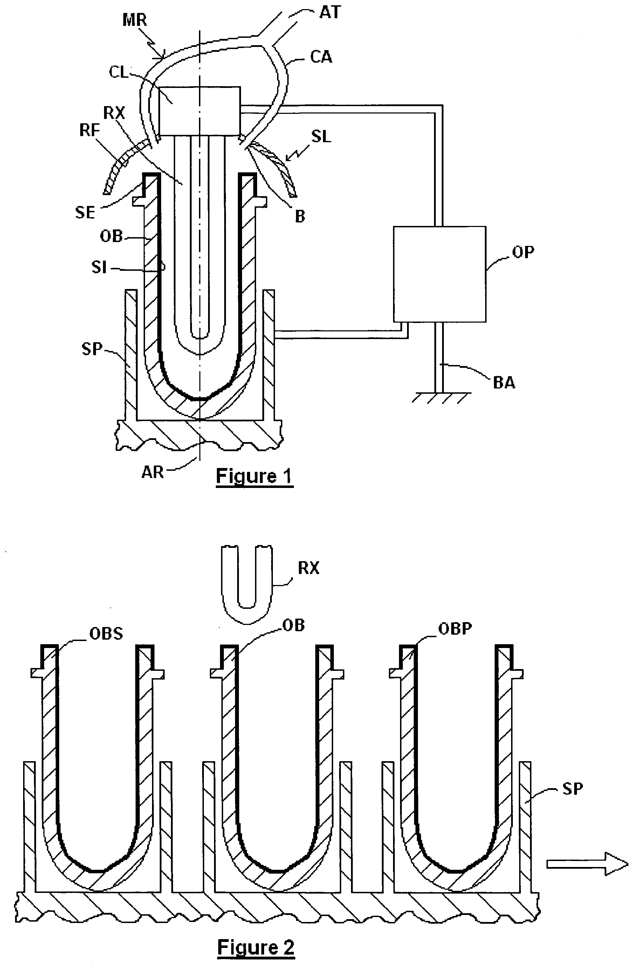

En référence à la figure 1, une source lumineuse SL comprend une lampe oblongue, de type xénon moyenne pression fonctionnant en mode pulsé, et un réflecteur RF. Cette lampe oblongue présente un corps rayonnant RX saillant par rapport à son culot CL, c'est-à-dire qu'une section plane sépare ce corps rayonnant lui-même oblong du culot portant les électrodes. Dans le cas présent, on adopte une lampe dite en U du fait de la forme du corps rayonnant RX.

Le réflecteur RF est fixé sur le culot CL.

Dans le cas présent, un objet creux OB présentant une cavité est une préforme de bouteille plastique positionnée et maintenue par un support d'insolation SP.

La préforme comprend une surface intérieure SI prévue pour être en contact avec un aliment et une surface extérieure SE prévue pour être en contact avec le bouchon de la bouteille.

Une décontamination de l'objet OB s'effectue tant dans la cavité (ici représentée par la surface intérieure SI) que sur la surface extérieure SE.

La lampe est positionnée, en position de travail, de telle sorte qu'une partie du corps rayonnant RX de cette lampe soit en saillie par rapport à l'objet OB.

L'illumination de la surface intérieure SI est effectuée par le rayonnement direct du corps rayonnant RX. On comprend bien qu'une lampe tubulaire dont les électrodes sont disposées aux extrémités d'un tube serait mal adaptée car la reprise de contact de son électrode disposée en regard du fond de la préforme produirait nécessairement une ombre lors de l'illumination. De plus, la connectique serait relativement complexe et l'ensemble lampe-connectique présenterait un encombrement conséquent.

L'illumination de la surface extérieure SE est effectuée par la réflexion de la lumière de la zone en saillie du corps rayonnant RX sur le réflecteur RF.

Ici, l'objet OB a un axe de symétrie de révolution AR. De ce fait, pour assurer d'efficacité du rayonnement, le réflecteur RF est de préférence une calotte sphéroïdale. L'axe du réflecteur RF coïncide avec cet axe de symétrie AR.

De plus l'axe formé par la grande dimension de la lampe est commun avec celui de la cavité. Ainsi, le corps rayonnant RX de la lampe peut pénétrer dans la cavité selon une trajectoire axiale suivant cet axe commun (ici représenté par l'axe de symétrie de révolution AR). De ce fait, l'axe de la calotte sphéroïdale du réflecteur RF, l'axe formé par la grande dimension de la lampe et l'axe de la cavité coïncident.

Bien entendu, ces trois axes sont communs car l'objet OB présente une symétrie de révolution. Cependant, la présente invention ne se limite pas à un objet OB dont la géométrie a une symétrie de révolution ; en conséquence ces différents axes peuvent être distincts.

Le dispositif est agencé sur un bâti BA, bâti symbolisé par une poutre.

Un organe de positionnement OP relie la source lumineuse SL au bâti BA. Cet organe de positionnement comprend des moyens pour déplacer axialement la lampe de sa position de travail (corps rayonnant RX pénétrant dans la cavité) à sa position de repos (corps rayonnant RX a l'extérieur de l'objet OB).

Cet organe consiste par exemple en une glissière actionnée par un vérin.

Le dispositif de décontamination comprend par ailleurs un moyen de refroidissement MR pour refroidir la source lumineuse SL. Par exemple, ce moyen de refroidissement MR envoie de l'air tempéré AT sur le corps rayonnant RX. Cet air tempéré AT provenant d'un circuit CA est projeté sur la lampe par des buses B situées par exemple au niveau du réflecteur RF.

En référence à la figure 2, un objet précédent OBP qui a déjà été décontaminé, l'objet en cours de traitement OB et un objet suivant OBS sont représentés. Le corps rayonnant RX a déjà illuminé l'objet précédent OBP. Il a également illuminé l'objet en cours OB et a donc été déplacé axialement de sa position de travail à sa position de repos par l'organe de positionnement OP. De ce fait, la lampe est à l'extérieur de la cavité de l'objet en cours OB. Puis, l'organe de positionnement OP comprend de plus des moyens pour déplacer transversalement le support d'insolation SP (direction symbolisée par la flèche dans la figure) de manière à axer la cavité de l'objet suivant OBS avec l'axe de la lampe. Ainsi, cet objet suivant OBS est positionné pour effectuer son illumination.

Il convient par ailleurs de rappeler que les lampes en quartz sont très fragiles. S'il advenait qu'une telle lampe casse au-dessus d'une préforme, celle-ci pourrait s'emplir de morceaux de quartz, ce qui n'est pas souhaitable. Pour pallier cet inconvénient, une solution consisterait à retourner tout le montage de sorte que les préformes soient disposées verticalement, ouvertures des cavités en bas, au-dessus de la lampe, disposée corps rayonnant en haut.

## Revendications

1. Dispositif de décontamination à source lumineuse d'un objet creux (OB) présentant une cavité et une surface extérieure (SE), comprenant une source lumineuse (SL), un support d'insolation (SP) et un organe de positionnement (OP), ladite source lumineuse (SL) comprenant une lampe oblongue présentant un corps rayonnant (RX) saillant par rapport à un culot (CL), ledit support d'insolation (SP) étant prévu pour positionner et maintenir ledit objet creux (OB), ledit organe de positionnement (OP) comprenant des moyens de positionnement axial pour produire un déplacement relatif de ladite source lumineuse (SL) par rapport audit support d'insolation (SP) selon leur axe commun entre une position de repos et une position de travail de sorte qu'en position de travail le corps rayonnant (RX) pénètre dans la cavité de l'objet creux (OB),
**caractérisé en ce que** la source lumineuse comprend en outre un réflecteur (RF) fixé sur ledit culot (CL) de telle sorte à permettre, en position de travail, l'illumination d'au moins une partie de la surface extérieure (SE) de l'objet (OB) par réflexion sur ledit réflecteur (RF) de la lumière d'une zone du corps rayonnant (RX) en saillie par rapport à l'objet (OB).

2. Dispositif de décontamination selon la revendication 1, **caractérisé en ce que** l'organe de positionnement (OP) comporte également des moyens pour produire un déplacement transversal entre le support d'insolation (SP) et la source lumineuse (SL), ce déplacement s'effectuant selon un axe distinct de leur axe commun.

3. Dispositif de décontamination selon l'une quelconque des revendications précédentes, **caractérisé en ce que** la plus grande dimension de la lampe (RX) coïncide avec l'axe commun.

4. Dispositif de décontamination selon l'une quelconque des revendications précédentes, **caractérisé en ce que** la source lumineuse (SL) est en service en position de travail et hors service en position de repos.

5. Dispositif de décontamination selon l'une quelconque des revendications précédentes, **caractérisé en ce qu'**il comprend en outre un moyen de refroidissement (MR) pour refroidir ladite source lumineuse (SL).

6. Dispositif de décontamination selon l'une quelconque des revendications précédentes, **caractérisé en ce que** le réflecteur (RF) est une calotte sphéroïdale.

7. Dispositif de décontamination selon l'une quelconque des revendications précédentes, **caractérisé en ce que** la lampe (RX, CL) est une lampe à xénon moyenne pression.

8. Dispositif de décontamination selon la revendication 7, **caractérisé en ce que** la lampe (RX, CL) fonctionne en mode pulsé.

9. Dispositif de décontamination selon l'une quelconque des revendications précédentes, **caractérisé en ce que** le corps rayonnant (RX) est en forme de « U ».

10. Utilisation d'un dispositif de décontamination selon l'une quelconque des revendications précédentes pour décontaminer un objet creux (OB) présentant une cavité, dans ladite cavité et sur une partie (SE) au moins de sa surface externe.

11. Utilisation selon la revendication 10, **caractérisé en ce que** l'objet creux (OB) est une préforme de bouteille plastique.

## Patentansprüche

1. Dekontaminationsvorrichtung mit einer Lichtquelle für die Dekontamination eines hohlen Objekts (OB), wobei die Dekontaminationsvorrichtung einen Hohlraum und eine äußere Oberfläche (SE), mit einer Lichtquelle (SL), eine Einstrahlungshalterung (SP) und eine Positionierungseinrichtung (OP) aufweist, wobei die Lichtquelle (SL) eine langgestreckte Lampe mit einem Leuchtkörper (RX) aufweist, der über einen Sockel (CL) herausragt, wobei die Einstrahlungshalterung (SP) dafür eingerichtet ist, das hohle Objekt (OB) zu positionieren und zu halten und wobei die Positionierungseinrichtung (OP) Mittel aufweist, um eine Relativbewegung der Lichtquelle (SL) relativ zu der Einstrahlungshalterung (SP) bezüglich ihrer gemeinsamen Achse zwischen einer Ruheposition und einer Arbeitsposition zu erzeugen, sodass der Leuchtkörper (RX) in der Arbeitsposition in den Hohlraum des hohlen Objekts (OB) eindringt, **dadurch gekennzeichnet, dass** die Lichtquelle weiterhin einen am Sockel (CL) angebrachten Reflektor (RF) aufweist, der dergestalt ausgebildet ist, um in der Arbeitsposition die Bestrahlung zumindest eines Teils der äußeren Oberfläche (SE) des hohlen Objekts (OB) durch Reflexion des Lichts eines von dem Objekt (OB) herausragenden Bereichs des Leuchtkörpers (RX) von dem Reflektor (RF) zu ermöglichen.

2. Dekontaminationsvorrichtung nach Anspruch 1, **dadurch gekennzeichnet, dass** die Positionierungseinrichtung (OP) weiterhin Mittel aufweist, um eine Transversalbewegung zwischen der Einstrahlungshalterung (SP) und der Lichtquelle (SL) zu erzeugen, wobei die Bewegung auf einer von ihrer gemeinsamen Achse verschiedenen Achse erfolgt.

3. Dekontaminationsvorrichtung nach einem der voranstehenden Ansprüche, **dadurch gekennzeichnet, dass** die größte Abmessung der Lampe (RX) mit der gemeinsamen Achse zusammenfällt.

4. Dekontaminationsvorrichtung nach einem der voranstehenden Ansprüche, **dadurch gekennzeichnet, dass** die Lichtquelle (SL) in der Arbeitsposition in Betrieb ist und in der Ruheposition außer Betrieb ist.

5. Dekontaminationsvorrichtung nach einem der voranstehenden Ansprüche, **dadurch gekennzeichnet, dass** sie weiterhin eine Kühlvorrichtung (MR) aufweist, um die Lichtquelle (SL) zu kühlen.

6. Dekontaminationsvorrichtung nach einem der voranstehenden Ansprüche, **dadurch gekennzeichnet, dass** der Reflektor (RF) eine sphäroite Kalotte ist.

7. Dekontaminationsvorrichtung nach einem der voranstehenden Ansprüche, **dadurch gekennzeichnet, dass** die Lampe (RX, CL) eine Xenon-Mitteldrucklampe ist.

8. Dekontaminationsvorrichtung nach Anspruch 7, **dadurch gekennzeichnet, dass** die Lampe (RX, CL) im gepulsten Modus betrieben wird.

9. Dekontaminationsvorrichtung nach einem der voranstehenden Ansprüche, **dadurch gekennzeichnet, dass** der Leuchtkörper (RX) in einer U-Form ausgebildet ist.

10. Verwendung einer Dekontaminationsvorrichtung nach einem der voranstehenden Ansprüche zur Dekontaminierung eines hohlen Objekts (OB) mit einem Hohlraum in dem Hohlraum und zumindest auf einem Teil seiner äußeren Oberfläche (SE).

11. Verwendung nach Anspruch 10, **dadurch gekennzeichnet, dass** das hohle Objekt (OB) ein Kunststoff-Flaschenvorformling ist.

## Claims

1. Decontamination device with a light source for a hollow object (OB) having
a cavity and an outer surface (SE), comprising a light source (SL), an exposure substrate (SP) and a positioning member (OP), said light source (SL) comprising an oblong lamp having a radiating body (RX) protruding with respect to a cap (CL), said exposure substrate (SP) being provided for positioning and holding said hollow object (OB), said positioning member (OP) comprising axial positioning means for producing a relative movement of said light source (SL) with respect to said exposure substrate (SP) along the common axis thereof between a rest position and a working position so that in the working position the radiating body (RX) enters the cavity of the hollow object (OB),
**characterized in that** the light source also comprises a reflector (RF) fixed on said cap (CL) so as to allow, in the working position, the illumination of at least a part of the outer surface (SE) of the object (OB) by reflection on said reflector (RF), of the light from a zone of the radiating body (RX) protruding with respect to the object (OB).

2. Decontamination device according to claim 1, **characterized in that** the positioning member (OP) also comprises means for producing a transverse movement between the exposure substrate (SP) and the light source (SL), this movement being made along an axis different from the common axis thereof.

3. Decontamination device according to any one of the preceding claims, **characterized in that** the largest dimension of the lamp (RX) coincides with the common axis.

4. Decontamination device according to any one of the preceding claims, **characterized in that** the light source (SL) is in service in the working position and out of service in the rest position.

5. Decontamination device according to any one of the preceding claims, **characterized in that** it also comprises a cooling means (MR) for cooling said light source (SL).

6. Decontamination device according to any one of the preceding claims, **characterized in that** the reflector (RF) is a spheroidal dome.

7. Decontamination device according to any one of the preceding claims, **characterized in that** the lamp (RX, CL) is a medium pressure xenon lamp.

8. Decontamination device according to claim 7, **characterized in that** the lamp (RX, CL) operates in pulsed mode.

9. Decontamination device according to any one of the preceding claims, **characterized in that** the radiating body (RX) is in the shape of a "U".

10. Use of a decontamination device according to any one of the preceding claims to decontaminate a hollow object (OB) having a cavity, in said cavity and on a part (SE) at least of the outer surface thereof.

11. Use according to claim 10, **characterized in that** the hollow object (OB) is a plastic bottle preform.
